# EUROPÄISCHE PATENTSCHRIFT

(11) **EP 1 132 416 B1**
(45) Veröffentlichungstag und Bekanntmachung des Hinweises auf die Patenterteilung: **28.04.2010**
(21) Anmeldenummer: 00104920.4
(22) Anmeldetag: 08.03.2000
(51) Int. Cl.: C08G 85/00, A61K 9/14

(54) **Kolloidale nanopartikuläre Träger enthaltend geladene oder ungeladene wasserlösliche Kammpolymere und deren Verwendung zur mucosalen Applikation**
Colloidal nanoparticular carriers comprising loaded or non-loaded water soluble comb polymers and their use in mucosal applications
Excipients de nanoparticles colloides comprenant des polymères en peigne chargés ou déchargés pour application locale par voie muqeuse

(43) Veröffentlichungstag der Anmeldung: 12.09.2001
(73) Patentinhaber: LAB International Barbados, SRL, Christ Church (BB)
(72) Erfinder: Kissel, Thomas, Prof. Dr., 79219 Staufen (DE); Breitenbach, Armin, Dr., 40789 Monheim (DE); Jung, Tobias, Dr., 52074 Aachen (DE); Kamm, Walter, Dr., 65719 Hofheim (DE)
(74) Vertreter: Buchhold, Jürgen

(56) Entgegenhaltungen:
- WO-A-99/52560
- DE-A- 19 839 515
- GB-A- 2 145 422
- US-A- 5 919 442
- US-A- 5 929 196
- BREITENBACH A ET AL: "Biodegradable comb polyesters: Part 1 Synthesis, characterization and structural analysis of poly(lactide) and poly(lactide-co-glycolide) grafted onto water-soluble poly(vinyl alcohol) as backbone" , POLYMER,GB,ELSEVIER SCIENCE PUBLISHERS B.V, VOL. 39, NR. 14, PAGE(S) 3261-3271 XP004119012 ISSN: 0032-3861 * das ganze Dokument *

## Beschreibung

Die vorliegende Erfindung hat zum Gegenstand einen kolloidalen Träger, welcher vorzugsweise in Form von Nanopartikel, als Trägerpolymer ein wasserlösliches amphiphiles Kammpolymer und zwar als Rückgrat (Backbone) mindestens ein wasserlösliches Polyol gepfropft (-g-) mit hydrophoben Seitenketten und ggf. ionischen Gruppen enthält. Die Erfindung betrifft vorzugsweise die Verwendung solcher kolloidalen partikulären Träger zur mucosalen Applikation.

Es besteht ein hohes Bedürfnis in der Bereitstellung neuer "Drug-delivery-Systeme" im Rahmen der modernen Pharmakotherapie. Wirkstoffformulierungen und Wirkstoffkombinationen werden immer wichtiger, die den Wirkstoff in eine applizierbare Form bringen und dabei besonders die Wirkstoffstabilität, dessen Bioverteilung, Bioverfügbarkeit und/oder Resorption positiv beeinflussen. Durch grosse Fortschritte in der Molekularbiologie, Gen- und Biotechnologie wird zunehmend eine immer grössere Anzahl an hydrophilen makromolekularen Wirkstoffen, wie z. B. Protein(oid)en, Nukleinsäure (-Konstrukten) samt Derivate und Wachstumsfaktoren, Vakzine, Impfstoffe etc. zugänglich. Allerdings ist die human- oder veterinärmedizinische Anwendung dieser Biomoleküle ohne geeignete Träger durch zahlreiche unerwünschte Nebenwirkungen erschwert bzw. sogar zum Teil nicht möglich.

Ganz besondere Anforderungen sind an geeignete Drug-delivery Systeme zu stellen, die in der Lage sind, sich an Schleimhäute (Mucosa) anzuheften (sogenannte Bioadhäsion) und diese oder MALT (mucosa associated lymphoid tissues) zu aktivieren und eine systemische Immunantwort zu induzieren. Als allgemein vorteilhaft gelten solche Systeme deren Träger bioabbaubar oder für eine chronische Applikation biokompatibel sind. Besonders vorteilhaft ist die Verwendung von Trägem deren Einzelkomponenten von bekanntermassen verträglichen Polymerklassen (also: biokompatibel) abgeleitet sind. Solche befähigte Systeme - im Rahmen einer mukosalen bzw. transmukosalen Applikation - sind in der Literatur bisher wenig beschrieben. Als allgemein vorteilhaft gelten solche Systeme aus oben beschriebenen Trägem in Form von Nanopartikeln (Carino, Mathiowitz, Adv. Drug Delivery Rev. 35(1999). 249-257; Allémann, Leroux, Gurny, Adv. Drug Delivery Rev. 34(1998), 171-189).

Die Druckschrift - Breitenbach, Kissel, Polymer 39, 3261, 1998 - offenbart ein Verfahren zur Herstellung von Kammpolymeren mit einem Polyvinylalkohol-Rückgrat (PVA-Backbone), welches mit hydrophoben alpha-Hydroxisäuren und zwar Poly(milch-co-glycolsäure) (kurz: PLG) unter Erhalt von PVA-g-PLG verpfropft wurde. Auf diesen Offenbarungsgehalt wird sich im Rahmen dieser Erfindung ausdrücklich bezogen.

Derartige Kammpolymere sind als vorteilhaft anzusehen, da sie eine hohe Biokompatibiltät sowie einen gewünschten amphiphilen Charakter aufweisen und unter bestimmtem Bedingungen wasserlöslich sein können.

In Breitenbach, Nykamp, Kissel, Self-assembling colloidal carriers for protein delivery: nanoparticulate polymer protein conjugates with novel watersoluble biodegradable comb polyolesters, Proc. Int. Symp. Control. Rel. Bioact. Mater. 26 (1999) 248 werden solche PVA-g-PLG Kammpolymere zu wasserlöslichen geladenen Sulfobutyl-PVA-g-PLG modifiziert.

Derartige geladene und ungeladene Kammpolymere (geladen: Kammpolyelektolyte) erweisen sich als geeignet in Gegenwart einer Proteinlösung einen kolloidalen Träger zu etablieren, welcher zur Selbst-Aggregation unter Erhalt von Nanopartikeln neigt. Die Steuerung der Selbst-Aggregation wird neben den Polymereigenschaften als pH und temperaturabhängig beschrieben.

Nachteilig ist jedoch, dass keine technische Lehre offenbart wird, die reproduzierbar solche vollständig wasserlösliche Polymere bereitstellt.

Überraschender Weise können aus solchen kolloidalen Träger Nanopartikel erhalten werden, welche besonders geeignet sind an die Mucosa anzulagem und diese oder MALT aufgrund ihrer besonders geeigneten Grösse und Ladung diese zu aktivieren.

Daher ist es die Aufgabe der Erfindung, einen kolloidalen nanopartikulären Träger, welcher als Trägerpolymer ein wasserlösliches Kammpolymer und / oder Kammpolyelektrolyten mit amphiphilen Charakter enthält bereitzustellen mit der Eigenschaft an die Mucosa oder MALT anzulagern, und diese oder MALT zu aktivieren und eine systemische Immunantwort zu induzieren.

Die Aufgabe wird dadurch gelöst, dass kolloidale nanopartikuläre Träger enthaltend ein wasserlösliches Kammpolymer bereitgestellt werden, wobei das wasserlösliche Kammpolymer ein Rückgrat aus mindestens einemwasserlöslichen Polyol sowie hydrophoben Seitenketten unter Ausbildung eines amphiphilen Charakters und ggf. ionischen Gruppen enthält, wobei das Rückgrat-Polymer ein mittleres Molekulargewicht (M(w)) von 10.000 bis 30.000 g/mol und samt hydrophoben Seitenketten entsprechend ein mittleres Molekulargewicht (M(w)) erzielt von vorzugsweise 45.000 bis 100.000 g/mol, und wobei die Tärger eine Größe von 10 bis 800 nm aufweisen.

In einer besonderen Ausführungsform ist das wasserlösliche Kammpolymer ein wasserlösliches Kammpolyelektrolyt mit einer zusätzlichen großen Zahl an ionischen dissoziierbarer Gruppen, die als integraler Bestandteil der Polymer-Hauptkette an diese seitlich (in den Kammzähnen) angehängt sind, neben den hydrophoben Seitenketten.

Ganz besonders bevorzugt ist daher ein vollständig lineares, wasserlösliches Kammpolymer und / oder Kammpolyelektrolyt enthaltend mindestens ein lineares wasserlösliches Polyol als Backbone mit einer mittleren molaren Masse (M(w)) von 10.000 - 30.000 g/mol, besonders bevorzugt 15000 - 25000 g/mol und ganz besonders bevorzugt 20.000 g/mol, welches mit hydrophoben Seitenketten ein Molekulargewicht (M(w)) erzielt von vorzugsweise 45000 - 100.000 g/mol, besonders bevorzugt 50000 - 80000 g/mol und ganz besonders bevorzugt 50.000 - 60.000 g/mol (Vergleiche hierzu Tabelle 3, dort wasserlösliche Beispiele solcher Kammpolymere).

Überraschender Weise werden aus den erhaltenen kolloidalen nanopartikulären Trägem der erfindungsgemäß verwendeten geladenen und ungeladenen wasserlöslichen linearen Kammpolymeren besonders geeignete Drug-delivery Systeme zur mucosalen Applikation erhalten.

Daher betrifft ein Gegenstand der Erfindung die besonders geeignete Bioadhäsion der kolloidalen nanopartikulären Träger an Schleimhäute und MALT mit der Fähigkeit eine systemische Immunantwort zu induzieren (siehe Beispiele).

Das Rückgrat-Polyol wird vorzugsweise aus der Klasse der dem Fachmann bekannten biokompatiblen Hydroxyl-Gruppen tragenden Polymere ausgewählt, wie nicht abschliessend genannt Polysaccharide, Polyalkohole, Polyvinylalkohol, Polyvinylacetat, Dextrane, ggf. Polyacrylate und jeweils entsprechende Derivate.

Erfindungsgemäss wird durch Aufpfropfen an das lineare Rückgrat-Polyol geeigneter hydrophober und zwar vorzugsweise bioabbaubarer oder biokompatibler Polymere ausgewählt, wie nicht abschliessend genannt aus Polylaktid, Polyglykolid, Poly(laktid-co-glykolid), Polytartrat die erfindungsgemässe Kammpolymer-Struktur erhalten.

Die Ausführung des Kammpolyelektrolyten wird durch Modifizierung des Kammpolymers mit geeigneten ionischen Gruppen erhalten ausgewählt, wie nicht abschliessend genannt Sulfobutyl-, Sulfopropyl-, Diethylaminoethyl-, Diethylaminomethyl-, Carboxyl-, Phosphat-, Sulfonsäure-Gruppen.

In einer besonderen Ausführungsform enthält der erfindungsgemässe Kammpolyelektrolyt ein PVA Rückgrat, mit belegten Seitenketten erhältlich aus 1,4-Butansulton, oder N-(2-chlorethyl)-N,N-diethyl-ammonium-chlorid bzw. N-(2-chlorethyl)-N,N-dipropyl-ammonium-chlorid und alpha-Hydroxisäuren wie Milchsäure und Glycolsäure bzw. aus deren dimeren Kondensationsprodukte.

Das Kammpolyelektrolyt trägt daher Elektrolyt-Funktionalitäten an jeder Wiederholungseinheit ihres Makromolekül.

Je nach Belegungsgrad der -OH Gruppen mit geladenen Gruppen (kationisch /anionisch) sind lonomere (weniger beladen) von Polysäuren und Polybasen (hoch beladen) zu unterscheiden. Die Wechselwirkung der Polyelektrolyte mit den Wirkstoffen kann sowohl über elektrotstatische als auch über hydrophobe Kräfte erfolgen.

Der erfindungsgemässe Polyelektrolyt liegt in Lösung infolge der intramolekularen elektrostatischen Abstossung der ionischen Gruppen meist als um ein Vielfaches stärker aufgeweitete Knäuelmolekül vor als man es von den ungeladenen Polymer-Molekül (Vgl. hier z.B. PVA-g-PLG) her kennt.

Als besonders vorteilhaft muss die Amphiphilität und die Wasserlöslichkeit der verwendeten wasserlöslichen Kammpolymeren in Bezug auf die potentiellen genannten Wirkstoffe gesehen werden. Die erfindungsgemässen Nanopartikel können durch einen überraschend einfachen Prozess ohne Einbringung von grossen Mengen mechanischer Energie oder hohen Scherkräften, insbesondere auch ohne die Verwendung von organischem Lösungsmittel , Tensiden, Emulgatoren oder anderen Hilfsmitteln vorteilhaft für die spätere Verträglichkeit der Anwendung gewonnen werden

Des weiteren betrifft die Erfindung ebenfalls ein Verfahren zur Herstellung des Polymerträgers, wobei ausgehend eines aktivierten wasserlöslichen Polyols, in einem ersten Schritt ggf. mit geladenen Gruppen beladen wird und in einem zweiten Schritt mit hydrophoben Seitenketten verbunden (z.B. Ester, Ether) wird (Vgl. Reaktionsschema 1 und Beispiel 1 und 2).

In einer bevorzugten Ausführungsform sind die geladenen Gruppen 1,4-Butansulfon (SB) oder N-(2-chlorethyl)-N,N-diethyl-ammonium-chlorid (DEAE). Es werden im ersten Schritt DEAE-PVA (kationisch) oder SB-PVA (anionisch) erhalten, welche im zweiten Schritt zum Kammpolymer mittels Poly(milch-co-glycolsäure) ausgebildet werden und zwar DEAE-PVA-g-PLG und SB-PVA-g-PLG.

Der zweite Schritt erfolgt vorzugsweise mittels Melt-grafting (Breitenbach supra) unter Schmelzpolymerisation unter Ringöffnung des Lactids und Glycolids.

Im Sinne dieser Erfindung können Nanopartikel des kolloidalen Trägers in unterschiedlicher Weise verabreicht (appliziert) werden, wie sublingual, subkutan, buccal, oral, nasal, pulmonal, vaginal, okular oder gastrointestinal.

Derartige Applikationen sind für alle pharmazeutisch relevanten, biologisch aktiven Stoffe von Interesse, wenn ein Wirkstofftransport an, in oder durch die Mukosa erwünscht ist (daher erfindungsgemäss: mucosale Applikation der genannten wasserlöslichen Kammpolymeren). Besonders vorteilhaft sind sie für Wirkstoffe, die ohne den Einsatz eines erfindungsgemässen kolloidalen Trägers bei sublingualer, buccaler, oraler, nasaler, pulmonaler, vaginaler, okularer oder gastrointestinaler Verabreichung zerstört oder inaktiviert werden, bzw. für die eine hohe lokale mukosale Konzentration (siehe Tetanus Toxoid Beispiel) erwünscht ist.

Der kolloidale Träger dient daher vorzugsweise als Wirkstoffträger. Als Wirkstoffe im Rahmen dieser Erfindung können nicht abschließend in Betracht gezogen werden:
1) Gruppe der hydrophilen makromolekularen pharmakologisch relevanten Wirkstoffe:
   a.) Impfstoffe, die ausgewählt werden aus der Gruppe: Lebend-Impfstoffe, Tot-Impfstoffe, Toxoid-Impfstoffe, DNA Konstrukte sowie deren adjuvierte Zubereitung ggf. gerichtet gegen pathogene Keime bzw. deren pathogenen Metaboliten, von denen literaturmässig der mukosale Penetrationsweg bekannt ist bzw. die vorwiegend auf mukosalen Oberflächen kolonisieren, wie z.B. Helicobacter pylori, typhiforme Salmonellen, Vibrio cholerae, Neisserien (N. gonorrhoee, N. meningitidis), Entamoeba histolytica, Haemophilus influenzae, Bordetella pertussis, Hepatitis A-Viren, colon- bzw. vagina-kolonisierende Staphylokokken, Streptokokken Typ A & B, Klebsiellen (K. pneumoniae, K oxytoca, K. rhinoscleromatis), Shigellen, Yersinien, Vibrionen, Pseudomonas aeroginosa, Legionellen, Korynebakterium diphtheriae, Bacillus anthracis, Mycobakterien (M. tuberkulosis, M. leprae), Treponema pallidum, Mycoplasma pneumoniae, Chlamydien (C. trachomatis, C. pneumoniae), Polio-Viren, Rhino-Viren, Myxoviren (Masern-V., Mumps-V., Parainfluenza-V.), Röteln-Virus, Rotaviren, Adeno-Viren, Influenzaviren, Herpes-Viren (Varizellen-Zoster-Virus, Zytomegalievirus, Herpes Simplex Viris, EBV), HAV, HEV;
   b.) Impfstoffe, die ausgewählt werden aus der Gruppe: Lebend-Impfstoffe, Tot-Impfstoffe, Toxoid-Impfstoffe, DNA Konstrukte sowie deren adjuvierte Zubereitung ggf. gerichtet gegen pathogene Keime bzw. deren pathogenen Metaboliten, bei denen bekanntermassen ein parenteraler Infektionsweg vorliegt, aber eine mukosale Applikation systemische, spezifische Immunglobulintiter hervorruft, wie z. B. Clostridium tetani, Clostridium botulinum, Yersinia pestis, Borrelia burgdorferi, Tollwut-Virus, HIV-1 & HIV-2, HBV, HCV, HDV;
   c.) Proteine, Peptide, Glyko- und Lipopeptide, wie z.B. Insulin, Calcitonin, Erythropoetin, Granulozyten Stimulierender Faktor (GcSF), Fibroblasten Stimulierender Factor (FGF), Parathyroidhormon (PTH), Somatostatin, Vertreter der Gruppe der Aminocandine, Ciclosporin.

Die Wirkstoffe können in wässriger Lösung in Gegenwart der genannten wasserlöslichen Kammpolymere versetzt werden, wobei in Selbst-Aggregation die vorteilhaften kolloidalen nanopartikulären Träger, enthaltend den gewünschten Wirkstoff, erhalten werden können.

Die Vorteile derartiger sich spontan aus wässrigen Lösungen ausbildenden Systemen liegen auf der Hand, u.a. sind keine besonderen Herstellungstechnologien nötig, es sind nicht zwingend, die Eigenschaften des Therapeutikums mindernden Hilfsstoffe, wie z.B. organische Lösungsmittel, Emulgatoren etc, notwendig.

Man erkennt, dass es besonders vorteilhaft ist, wenn die kolloidalen nanopartikulären Träger Grösse von 10 bis 800 nm aufweisen, vorzugsweise kleiner als 500 nm, besonders bevorzugt im Bereich von 30 - 200 nm.

Die zu erzielende Größe ist abhängig von Größe, Ladung und Flexibilität, Elastizität des ausgewählten Wirkstoffes und des ausgewählten geladenen ungeladenen Kammpolymers. Grössen im Bereich kleiner 200 nm sind besonders geeignet für die mucosale Applikation.

Dabei wurde gefunden, dass sich der Größenbereich von unter 500 nm Durchmesser erfindungsgemäß besonders gut zur mukosalen Applikation eignet, da sich in überraschender Weise eine besonders erhöhte Wirkstoffkonzentration auf relevanten Zellen einstellte, sowie in vivo eine entsprechende systemische Immunantwort induzieren lässt (vgl. Florence, The oral absorption of micro- and nanoparticulates: neither exceptional nor unusual. Pharm. Res. (1997), 14(3), 259-266 und Florence; Hillery; Hussain; Jani, P.U.; Oral uptake and translocation of nanoparticles: a real but useful phenomenon? NATO ASI Ser., Ser. A (1994), Volume Date 1994, 273 173-81.).

Wegen der einerseits weit variablen, andererseits sehr spezifisch einstellbaren Eigenschaften zeigen die erfindungsgemäßen kolloidalen nanopartikulären Träger Eigenschaftsprofile, die sie insbesondere auch für eine mukosale Applikation prädestinieren. In der Literatur ist bekannt, dass der Transport von Partikeln größenabhängig ist, die erfindungsgemäßen Nanopartikel liegen in einem Bereich, der für den Transport allgemein als günstig erachtet wird. Die erfindungsgemäßen Kammpolyelektrolyte können positiv geladen sein - die Schleimhäute negativ - und bewirken daher eine bessere Bioadhäsion.

Weitere Vorteile, die sich durch die Erfindung ergeben, sind die für den Patienten angenehmere Art der Applikation, da nicht zwingend parenteral, also über eine Injektion, appliziert werden muss, und die damit verbundene erhöhte 'Patienten'-Compliance. Hierdurch kann, gegenüber bisherigen Verfahren bei der Applikation von Protein- und Peptid-Wirkstoffen oder Impfverfahren, eine therapeutische Wirkung des Proteins oder eine Schutzimpfung z.B. durch Einnahme einer Tablette oder Lösung, ähnlich der oralen Verabreichung von Ciclosporin oder der Poliomyelitis-Impfung (Kinderlähmung), erfolgen.

### Beispiele

Die Beispiele dienen zur näheren Erläuterung der Erfindung, ohne diese auf die Beispiele zu begrenzen.

Materialien: PVA mit einem Molekuargewicht von 15.000 g/mol und einem Hydroylsegrad von 88% wurde bei Fluka erworben. Vor dem Gebrauch wurde die PVA bei 80°C im Vakuum bis zum konstanten Gewicht getrocknet. D, L Lactid und Glycolid (Boehringer Ingelheim) wurden zweimal in trockenem Ethylacetat rekristallisiert (Rückfluss über Kalziumhydrid) und 48 h im Vakuum getrocknet. Stannous octoate Zinnoctoat(Aldrich), 1,4 Butanesultone (purum, Fluka), N-(2-chlorethyl)-N,N-diethyl-ammonium-chlorid (Merck-Suchard) und andere Materialien wurden in analytischer Reinheit gehalten verwendet. Zur Herstellung der erfindungsgemässen Polymere wird auf die Schrift Breitenbach, Kissel Polymer 39, 3261, 1998 verwiesen.

### Beispiel 1

### Polymersynthese:

### Herstellung des Polyelektrolyten:

Sulfobutyl-PVA (SB(xx)-PVA) und Diethylaminoethyl-PVA (DEAE(xx)-PVA) wurden hergestellt aus PVA und zwar unter den nicht wässrigen Bedingungen nach Williamson in einer trockenen Stickstoffatmosphäre (Vgl. Dolle F, Le Moigne J, Gramain P. Etherification de l'alcool polyvinylique-I. Reaction avec la propanesultone. Eur. Polym. J. 1970; 6: 1227 und Gramain P, Le Moigne J. Etherification de l'alcool polyvinylique-II. Preparation de derives amphipathiques par alcoylation et sulfopropylat. Eur. Polym. J. 1972; 8: 703). (xx) bezzeichnet den Substitutionsgrad an OH Gruppen in mol%.

**Tabelle 1**

| Bezeichnung | Molekulargewicht [kg/mol] | Substitutionsgrad [%] | Substituent an -OH |
|---|---|---|---|
| PVA | ∼ 6 (*) | (20) | (-O-COCH3) |
| PVA | ∼ 15 (*) | (12) | (-O-COCH3) |
| SB03PVA | ∼ 16 | 3 | -O-(CH2)4SO3- |
| SB10PVA | ∼ 19 | 10 | -O-(CH2)4SO3- |
| SB20PVA | ∼ 23 | 20 | -O-(CH₂)₄SO₃⁻ |
| SB30PVA | ∼ 27 | 30 | -O-(CH₂)₄SO₃⁻ |
| SB40PVA | ∼ 31 | 40 | -O-(CH₂)₄SO₃⁻ |
| SB50PVA | ∼35 | 50 | -O-(CH₂)₄SO₃⁻ |
| DEAE10PVA | ∼18 | 10 | -O-(CH₂)₂N(C₂H₅)₂ |

| | | | |
|---|---|---|---|
| * Herstellerangabe | | | |

Es wurden 2,4 g (0,1 mol) gereinigtes Natriumhydrid in 50 ml trockenem DMSO bei 20 DEG C in Eiskühlung unter Rühren eingegeben bis keine Gasentwicklung mehr beobachtet wurde. Das erhaltene Carbanion des DMSO wurde tropfenweise innerhalb einer Stunde mit einer 5g PVA Lösung in 100 ml trockenem DMSO bei RT versetzt. Nah einer Stunde Reaktion unter Eiskühlung wurden 13, 6 g (0,1 mol) 1,4-Butansulton oder 17, 3 g (0,1 mol) N-(2-chlorethyl)-N,N-diethyl-ammoniumchlorid in 20 ml trockenem DMSO innerhalb 1,5 h zugetropft. Das Produkt wurde in 70: 30 (v/v) Aceton / Hexan aufgenommen und im Vakuum bis zur Gewichtskonstanz getrocknet. Anschließend wurde das getrocknete Produkt mittels Ultrafiltration (Amicon 8010, YM1 Filtermembran) aufgereinigt.

Melt Grafting zur Herstellung des erfindungsgemäßen Trägers mittels Pfropfung von alpha-Hydroxycarbonsäurekondensate an die Zentralpolyole Beispielhaft für die Lactone der Milch- und Glycolsäure: D,L-Lactid (LA: aus intermolekularer Wasserabspaltung von 2 Teilen Milchsäuren) und Glycolid (GA: entsprechend aus Glycolsäure) werden in definierten molaren Mengen, z.B. 1 : 1, in Gegenwart definierter Mengen des Zentralpolyols, z.B. 1 Gew% PVA, bei z.B. 130 DEG C für z.B. drei Stunden unter inerten, wasserfreien Bedingungen mit definierten molaren Mengen Katalysator (z.B. 0.2 mmol Zinnoctoat) umgesetzt. Anschliessend wird das Reaktionsprodukt in einem geeigneten Lösungsmittel (Dichlormethan: DCM) aufgenommen und in einem 10:1 Überschuss eines damit mischbaren Polymemichtlösungsmittels (Ethanol) zur Aufreinigung ausgefällt und bis zur Gewichtskonstanz im Vakuum getrocknet (Tabelle 2).

**Tabelle 2**

| Polymer | D,L-LA:GA [mol:mol] | Zentralpolyol*/menge [Gew.%] | Reaktionstemp. [°C] | Lösungs-Mittel |
|---|---|---|---|---|
| PVAPLG1 | 1:1 | PVA/1 | 130 | DCM |
| PVAPLG 10 | 1:1 | PVA/10 | 130 | Aceton |
| PVAPLG 33 | 1:1 | PVA/33 | 140 | Aceton/Wasser |
| PVAPLG 50 | 1:1 | PVA/50 | 140 | Wasser |
| PVAPLG 57.1 | 1:1 | PVA/57,1 | 140 | Wasser |
| PVAPLG 62.5 | 1:1 | PVA / 62,5 | 140 | Wasser |
| PVAPLG 70 | 1:1 | PVA170 | 140 | Wasser |
| PVAPLA5 0 | 1*:0(*L-LA) | PVA/50 | 110 | Wasser |
| SB03PVA PLG10 | 1:1 | SB03PVA/10 | 140 | Aceton |
| SB10PVA PLG10 | 1:1 | SB10PVA/10 | 140 | Aceton |
| SB20PVA PLG10 | 1:1 | SB20PVA/10 | 140 | Aceton |
| SB30PVA PLG10 | 1:1 | SB30PVA/1 | 140 | Aceton |
| SB40PVA PLG10 | 1:1 | SB40PVA/10 | 140 | Aceton |
| SB50PVA PLG10 | 1:1 | SB50PVA/10 | 140 | Aceton |
| DEAE10 PVAPLG 10 | 1:1 | DEAE10PVA/10 | 150 | Aceton |
| SB40PVA PLG33 | 1:1 | SB40PVA/33 | 140 | Aceton/Wasser |
| SB40PVA PLG50 | 1:1 | SB40PVA/50 | 140 | Wasser |
| SB10PVA PLG45 | 1:1 | SB10PVA/45 | 140 | Wasser |
| SB10PVA PLG50 | 1:1 | SB10PVA/50 | 140 | Wasser |

| | | | | |
|---|---|---|---|---|
| Reaktionszeit jeweils 3 Stunden, Katalysator jeweils Zinnoctoat * entsprechend Tabelle 1 | | | | |

### Beispiel 2

### Charaktersierung der Polymere

2.1 Grössenausschluss-Chromatographie (SEC) und statische Lichtstreuung (SLS) 0.5% (mN) ige Polymerlösungen wurden in ein auf 35 DEG C thermostatiertes Merck-Hitachi Chromatographiesystem (Säulen: Lichrogel PS mix und Lichrogel PS 40, 10 µm) mit Differential-Refraktometer (RI71) und einem MiniDawn Lichtstreudetektror (Wyatt Technology Corp.) injiziert. (100 µl K5 Zelle, Wellenlänge Laserlicht 690 nm, Laser Power 30 mW, Detektion bei 45 DEG , 90 DEG and 135 DEG).

### Beispiel 3

Adhäsions- und Aufnahmestudien der Nanopartikel im Zellkulturmodell Generelle Methodenbeschreibung Zellversuche Bestimmung der Zytoadhäsion und Zytotoxizität

Voraussetzung für eine mucosale Applikation, ist neben einer nur geringen toxischen Wirkung auf das umgebende Gewebe (Epithelzellen), auch eine ausreichend lange Verweilzeit auf mukosalen Oberflächen (Zytoadhäsion) sowie eine eventuell erforderliche ausreichende zelluläre Aufnahme. Zur Untersuchung der Wechselwirkung mit mucosalen Zellen wurde das Caco 2 Zellkulturmodell herangezogen. Dieses in vitro -Modell der intestinalen Mucosa hat bereits in vielen Untersuchungen seine Eignung zum Studium von intestinaler Resorption, Cytoadhäsion und Toxizität von Arzneistoffen und Arzneistoffträgersystemen zeigen können.

### Zellkultur:

Caco 2 Zellen der Passagen 42 bis 45 wurden kultiviert in Dulbecco Modified Eagles-Medium mit einem Glucosegehalt von 4.5g/l unter Zusatz von 10% Fötalem Kälberserum, 2mM L-Glutamin und 1% Nichtessentieller Aminosäuren in 10% iger CO2 -Atmosphäre bei 95% RF und 37 DEG C.

### Toxizitätsuntersuchungen:

Caco 2 Zellen wurden in einer Zelldichte von 6.5 x 104 Zellen / cm2 auf Polystyrol-Multiwell-Platten ausgesät. Mediumwechsel erfolgte alle 2 Tage. Am Tag 21 nach Aussaat haben die Zellen eine differenzierte Monozellschicht ausgebildet und sind für in vitro Untersuchungen nutzbar. Hierzu wurden Zellen 2x gewaschen mit isotonischer phosphatgepepufferter Kochsalzlösung (PBS, pH 7.2) und entsprechende erfindungsgemässen Kolloid-Suspensionen (0.25 , 2.5 mg/ml) auf die Zelloberfläche aufgebracht. Nach mehrstündiger Inkubation (Zeitintervalle gemäss Abbildungen) wurden die nachfolgend genannten Toxizitätsprüfungen durchgeführt.
1.) Freisetzung von Lactatdehydrogenase (LDH): Prinzip: Als cytoplasmatisches Enzym tritt LDH bei einer Schädigung der Plasmamembran aus dem Zellinnern in das Aussenmedium (Puffer) über und kann mittels kinetischer Bestimmungsmethoden gegen einen Standard UV-photometrisch quantifiziert werden. Als toxischer Referenz-Standard dient Triton X 100 (0.1% ig in PBS), dessen LDH-Freisetzung 70% entspricht.
2.) Transformation von MTT (3-(4,5-dimethylthiazol-2-yl)-2,5-diphenyl tetrazolium bromide) Prinzip: Die Tetrazolverbindung MTT wird in Mitochondrien noch lebensfähiger Zellen zu einem blaugefärbten Formazanfarbstoff enzymatisch reduziert. Die Blaufärbung ist dabei proportional zur Viabilität der Zellen und wird photometrisch erfasst, als untoxische Referenz dient PBS.
3.) Propidium-lodid-FärbungPrinzip: Propidiumiodid durchdringt als hydrophile Substanz nur geschädigte Zellmembranen und bindet an Nukleinsäuren des Kerns, wodurch es bei UV-Anregung zu einer roten Kernfluoreszenz kommt, die mikroskopisch erfassbar ist. Die Auswertung erfolgt fluoreszenzmikroskopisch mit Wellenlängerfilter (380/550) gegen einen untoxischen PBS-Standard.
4.) Zytoadhäsion und zelluläre Aufnahme Caco 2 Zellen wurden in einer Zelldichte von 6.5 x 104 Zellen/cm2 auf Polycarbonatmembranen (Costar-Transwell) ausgesät. Mediumwechsel erfolgte alle 2 Tage. Am Tag 21 nach Aussaat haben die Zellen einen differenzierten Monolayer ausgebildet und können für Adhäsions-und Aufnahme-Untersuchungen verwendet werden.
5.) Mit Nilrot (1%,w/w) fluoreszenzmarkierte Kolloide sowie Polymer-Wirkstoff-Komplexe (Wirkstoff: mit Fluorescein-lsothiocyanat markiertem Rinderserumalbumin (FITC-BSA)) wurden auf die apikale Oberfläche der Zellen appliziert und für 120 min bei 37°C, 95% RF, 10% CO2 inkubiert. Die Kolloidkonzentration betrug in beiden Fällen 250pg/ml. Nach Inkubation wurden die Zellmonoschichten 3 mal mit PBS gewaschen und anschliessend mit Formalinlösung (4%ig in PBS) 60 min lang fixiert. Die Filtermembranen wurden mittels Skalpell herausgeschnitten und in Glycerol-Gelatine eingebettet. Die so hergestellten Präparate wurden mittels Konfokaler Laser Scanning Mikroskopie (CLSM) auf adhäsive und intracytoplasmatische Fluoreszenz untersucht.

Bei Kontrollen nur mit Wirkstoff ohne Kolloide bzw. Komplexe war keine Fluoreszenz auf bzw. in den Zellen nachweisbar.

### Beispiel 4

Female Balb/c mice, 7-9 Wochen alt, Gewicht 16-22 (erhältlich bei Harlan-Winkelmann, Marburg, Deutschland). 5 Gruppen wurden vergleichend eingesetzt. Kolloide Dispersionen wurden verglichen sowohl mit konventionellen Alumabsorbierten als auch freien Tetanus Toxoid (Ttx). Mäuse wurden randomisiert, gruppiert in Gruppen ä 10 Tieren und immunisiert in drei konsekutiven Wochen (Tag 1, 8, 15) in peroraler (p.o.) Applikation von 200 µl 5LF Ttx, welche die kolloidalen Polyelectrolyte enthielten. Die i.p. Inokulation (200 µl of Tetanol TM ) wurde in einer Positivkontrolle durchgeführt. Eine Abnahme erfolgte in Woche 0 und 4. Alle Seren wurden im ELISA Assay für Ttx spezifische IgG and IgA Antikörper durchgeführt. Verdünnungsreihe erfolgte in beschichteten TTF 6 Mikrotiter Platten. Ttx spezifische Antikörper wurden quantifiziert in Inkubation mit heavy chain-spezifischen Peroxidasen konjugierten Ziege anti Maus IgG oder IgA. (Messung bei (OD) 450 nm in Inkubation mit TMB). Die Ergebnisse wurden ausgewertet als reziproke End-Punkt Seren Titer mit höchster gegebener Serum Dilution: OD Wert 0.2.

Die Bioadhäsion in Maus wurde untersucht mittels Tetanus Toxoid (Ttx) als Modell Antigen. Drei Dosen 5 LF des Ttx wurden im Verlauf von drei Wochen (n=10) oral verabreicht.

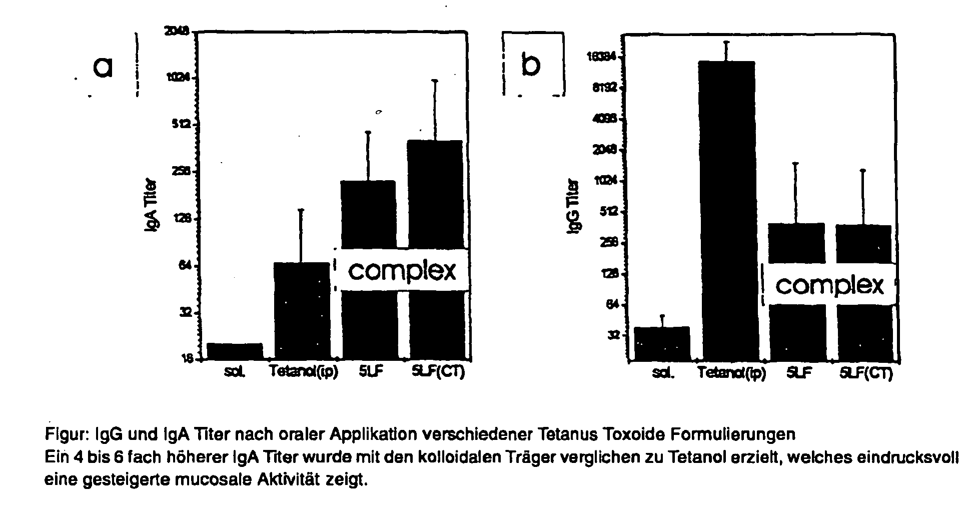

Figur: IgG und IgA Titer nach oraler Applikation verschiedener Tetanus Toxoide Formulierungen Ein 4 bis 6 fach höherer IgA Titer wurde mit den kolloidalen Träger verglichen zu Tetanol erzielt, welches eindrucksvoll eine gesteigerte mucosale Aktivität zeigt.

**Tabelle 3: geladene / ungeladene Kammpolymere (beachtlich sind die jedoch nur die Wasserlöslichen)**

| No | Polymer | PVA DS a) [%]/[mass%] | Backbone Mw a) [g/mol] | PLG Kette Mn b) [g/mol] | PLG Einheiten Pro Kette b) | Polymer Mn b) [g/mol] | LA:GA b) [mol%] | Lsgm. |
|---|---|---|---|---|---|---|---|---|
| 1 | PVA-g-PLGA | - | 15'000 | 4000 | 32 | 1'250'000 | 51 : 49 | DiChlorMethan |
| 2 | PVA-g-PLGA | - | 15'000 | 1100 | 9 | 360'000 | 51:49 | 1:1 (DCM : aceton) |
| 3 | PVA-g-PLGA | - | 15'000 | 590 | 5 | 238'000 | 50:50 | Aceton |
| 4 | PVA-g-PLGA | - | 15'000 | 390 | 3 | 134'000 | 50 : 50 | Aceton |
| 5 | PVA-g-PLGA | - | 15'000 | (50)* | (0.8)* | (30'000)* | (50: 50)* | Wasser |
| 6 | PVA-g-PLGA | - | 15'000 | (37)* | (0.4)* | (26'000)* | (50:50)* | Wasser |
| 7 | PVA-g-PLGA | - | 15'000 | (30)* | (0,3)* | (24'000)* | (50:50)* | Wasser |
| 8 | PVA-g-PLGA | - | 15'000 | (21)* | (0.2)* | (21'000)* | (50 : 50)* | Wasser |
| 9 | P(SB-VA)-g-PLGA | 14/S=6.8 | 19'900 | 590 | 5 | 172'000 | 53 : 47 | Aceton |
| 10 | P(SB-VA)-g-PLGA | 14/S=6.8 | 199'900 | (50)* | (0,4)* | (33'000)* | (50 : 50)* | Wasser |
| 11 | P(SB-VA)-g-PLGA | 27/S=10.0 | 26'000 | 840 | 7 | 210'000 | 52 : 48 | Aceton |
| 12 | P(SB-VA)-g-PLGA | 27/S=10.0 | 26'000 | (120)* | (2)* | (52'000)* | (50 : 50)* | 1:1 (Wasser : Aceton) |
| 13 | P(SB-VA)-g-PLGA | 27/S=10.0 | 26'000 | (60)* | (0.5)* | (35'000)* | (50 : 50)* | Wasser |
| 14 | P(SB-VA)-g-PLGA | 43/S=12.3 | 33'600 | 1100 | 9 | 221'000 | 53 : 47 | Aceton |
| 15 | P(SB-VA)-g-PLGA | 43/S=12.3 | 33'600 | (80)* | (0.6)* | (35'000)* | (50 : 50)* | Wasser |

| | | | | | | | | |
|---|---|---|---|---|---|---|---|---|
| a) = aus Elementaranalyse b) = aus NMR nach Standardmethoden | | | | | | | | |

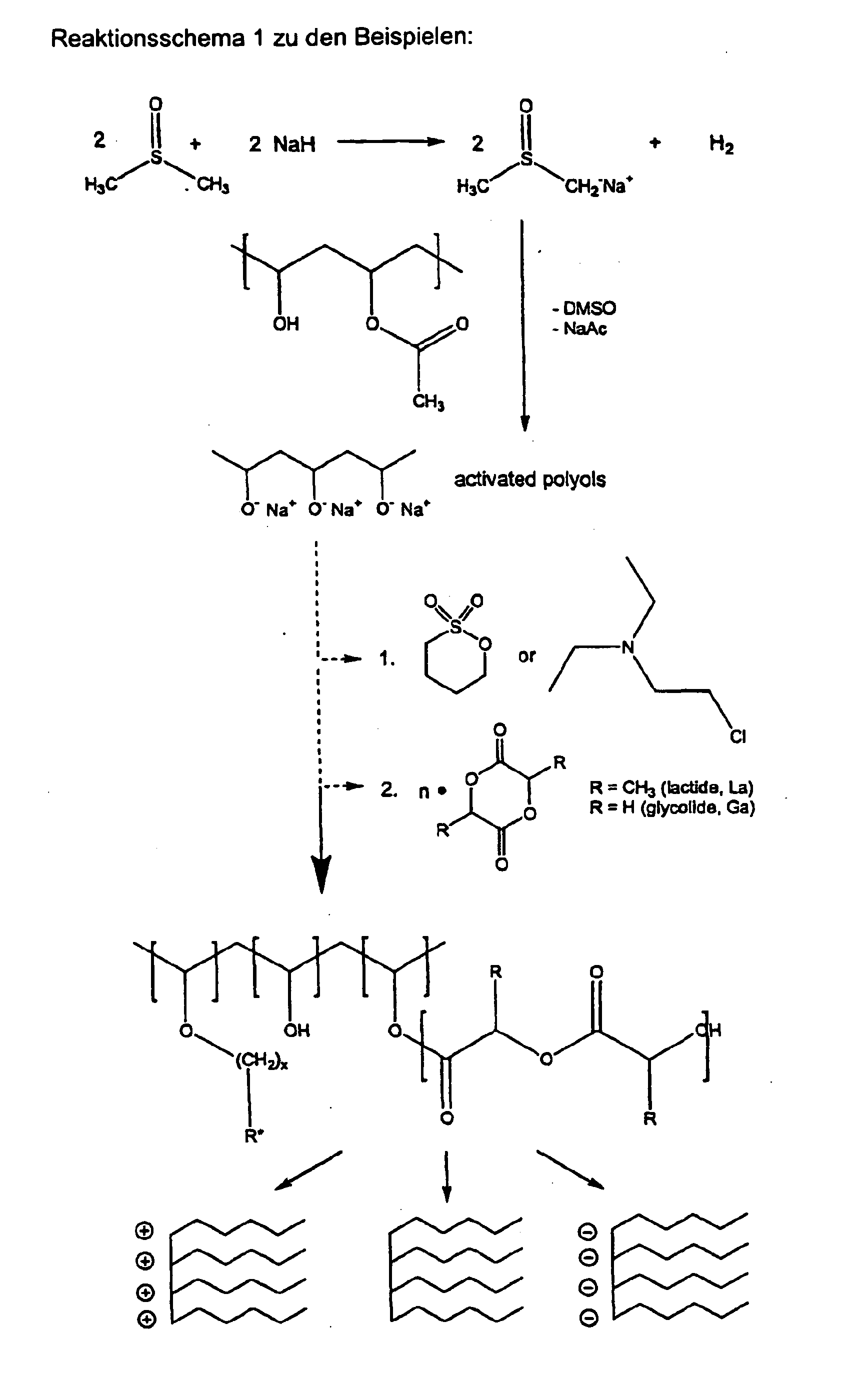

## Patentansprüche

1. Kolloidaler nanopartikulärer Träger enthaltend ein wasserlösliches Kammpolymer, wobei das wasserlösliche Kammpolymer ein Rückgrat aus mindestens einem wasserlöslichen Polyol sowie hydrophoben Seitenketten unter Ausbildung eines amphiphilen Charakters und ggf. ionischen Gruppen enthält, wobei das Rückgrat-Polymer ein mittleres Molekulargewicht (M(w)) von 10.000 - 30.000 g/mol und samt hydrophoben Seitenketten entsprechend ein mittleres Molekulargewicht (M(w)) von vorzugsweise 45000 - 100.000 g/mol erzielt, und wobei die Träger eine Größe von 10 bis 800 nm aufweisen.

2. Kolloidaler nanopartikulärer Träger nach Anspruch 1, **dadurch gekennzeichnet, dass** das wasserlösliche Polyol ausgewählt ist aus der Gruppe Polysaccharide, Polyalkohol, Polyvinylalkohol, Polyvinylacetat und Dextrane.

3. Kolloidaler nanopartikulärer Träger nach Anspruch 1 oder 2, **dadurch gekennzeichnet, dass** die hydrophobe Seitenkette ausgewählt ist aus der Gruppe Polylaktid, Polyglykolid, Poly(laktid-co-glykolid) und Polytartrate.

4. Kolloidaler nanopartikulärer Träger nach einem der Ansprüche 1 bis 3, **dadurch gekennzeichnet, dass** das Rückgrat-Polymer (M(w)) ein mittleres Molekulargewicht von 15000 - 25000 g/mol und bevorzugt 20.000 g/mol, und samt hydrophoben Seitenketten entsprechend ein mittleres Molekulargewicht (M(w)) erzielt von 50000 - 80000 g/mol und besonders bevorzugt 50.000 - 60.000 g/mol.

5. Kolloidaler nanopartikulärer Träger nach einem der Ansprüche 1 bis 4, **dadurch gekennzeichnet, dass** dieser an der Mucosa eine systemische Immunantwort induziert.

6. Verwendung eines kolloidalen nanopartikulären Trägers nach einem der Ansprüche 1 bis 5 enthaltend mindestens ein wasserlösliches Kammpolymer zur mucosalen Applikation.

## Claims

1. Colloidal nanoparticulate carrier containing a water-soluble comb polymers, with the water-soluble comb polymer containing a backbone of at least one water-soluble polyol and hydrophobic side chains forming an amphiphil character and, and if so, ionic groups, with the backbone polymer reaching a mean molecular weight (M(w)) of 10.000 - 30.000 g/mol, and with the hydrophobic side chains a respective mean molecular weight (M(w)) of preferably 45000 - 100.000 g/mol, and with the carriers showing a size of 10 to 800 nm.

2. Colloidal nanoparticulate carrier according to Claim 1, **characterized in that** the water-soluble polyol is chosen from the polysaccharides, polyalcohol, polyvinyl alcohol, polyvinyl acetate and dextranes group.

3. Colloidal nanoparticulate carrier according to Claim 1 or 2, **characterized in that** the hydrophobic side chain is chosen from the polylactides, polyglycolide, poly(lactide-co-glycolide) and polytartrates group.

4. Colloidal nanoparticulate carrier according to one of the Claims 1 to 3, **characterized in that** the backbone polymer (M(w)) reaches a mean molecular weight of 15000 - 25000 g/mol and preferably of 20.000 g/mol, and with the hydrophobic side chains a respective mean molecular weight (M(w)) of 50000 - 80000 g/mol, especially preferably of 50.000 - 60.000 g/mol.

5. Colloidal nanoparticulate carrier according to one of the Claims 1 to 4, **characterized in that** the same induces a systemic immunoresponse at the mucosa.

6. Use of a colloidal nanoparticulate carrier according to one of the Claims 1 to 5 containing at least one water-soluble comb polymer for mucosal application.

## Revendications

1. Porteur colloïdal nanoparticulaire contenant un polymère peigne soluble dans l'eau, le polymère peigne contenant une colonne vertébrale d'au moins un polyol soluble dans l'eau et des chaînes latérales hydrophobes formant un caractère amphiphil, et des groupements ioniques, le polymère à colonne vertébrale atteignant un poids moléculaire moyen (M(w)) de 10.000 - 30.000 g/mol et avec les chaînes latérales hydrophobes un poids moléculaire moyen (M(w)) respectif de 45000 - 100.000 g/mol de préférence, avec les porteurs montrant une dimension de 10 à 800 nm.

2. Porteur colloïdal nanoparticulaire selon la Revendication 1, **caractérisé en ce que** le polyol soluble dans l'eau est choisi du groupement polysaccharides, polyalcool, polyvinylalcool, polyvinylacétate, et dextranes.

3. Porteur colloïdal nanoparticulaire selon la Revendication 1 ou 2, **caractérisé en ce que** la chaîne latérale hydrophobe est choisi du groupement polyactide, polyglycolide, poly(lactide-co-glycolide), et polytartrates.

4. Porteur colloïdal nanoparticulaire selon une des Revendications 1 à 3, **caractérisé en ce que** le polymère à colonne vertébrale (M(w)) atteint un poids moléculaire moyen de 15000 - 25000 g/mol, et de préférence de 20.000 g/mol, et avec les chaînes latérales hydrophobes un poids moléculaire moyen (M(w)) respectif de 50000 - 80000 g/mol, et plus préférablement de 50.000 - 60.000 g/mol.

5. Porteur colloïdal nanoparticulaire selon une des revendications 1 à 4, **caractérisé en ce que** celui-ci induit une immunoréponse systémique.

6. Emploi d'un porteur colloïdal nanoparticulaire selon une des Revendications 1 à 5 contenant au moins un polymère peigne soluble dans l'eau pour application mucosale.
